(19) Europäisches Patentamt
European Patent Office
Office européen des brevets

(11) EP 1 008 847 A2

(12) EUROPÄISCHE PATENTANMELDUNG

(43) Veröffentlichungstag:
**14.06.2000 Patentblatt 2000/24**

(51) Int. Cl.[7]: **G01N 27/12**

(21) Anmeldenummer: **99123914.6**

(22) Anmeldetag: **01.12.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **07.12.1998 DE 19856369**

(71) Anmelder:
**SIEMENS AKTIENGESELLSCHAFT
80333 München (DE)**

(72) Erfinder:
- **Kornely, Susanne
80689 München (DE)**
- **Seidl, Monika, Dr.
80689 München (DE)**
- **Meixner, Hans, Prof.
85540 Haar (DE)**
- **Fleischer, Maximilian, Dr.
85635 Höhenkirchen (DE)**
- **Lampe, Uwe, Dr.
21614 Buxtehude (DE)**
- **Mrotzek, Christine
85560 Ebersberg (DE)**
- **Pohle, Roland
85570 Herdweg (DE)**
- **Giber, Janos, Prof.
1136 Budapest (HU)**

(54) **Resistiver Gassensor und Verfahren zu dessen Herstellung**

(57) Zur Messung von Stickoxiden, Ammoniak und Kohlenwasserstoff wird ein resistiver Gassensor beschreiben, dessen gassensitive Schicht aus einem Gemisch von $WO_3/TiO_2 \geq 50$ Gew.-% besteht. Hierdurch wird ausreichende Sensitivität und Temperaturstabilität für beispielsweise Messungen im Abgastrakt eines Fahrzeuges bewirkt. Die gassensitive Schicht wird naßchemisch dargestellt. In einer Variante wird das Wolframtrioxid als getragene Verbindung dargestellt. In einer anderen Variante wird in einem Sol-Gel-Verfahren ein Makromolekül aus dem Gemisch dargestellt.

FIG 4

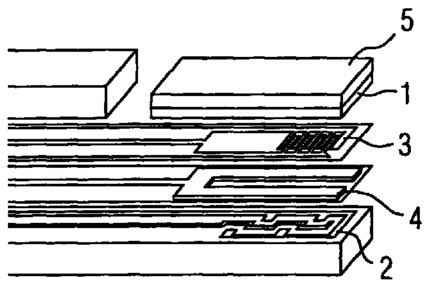


EP 1 008 847 A2

## Beschreibung

Die Erfindung betrifft einen resistiven Gassensor mit einer gassensitiven Schicht deren Widerstand sich in Anwesenheit bestimmter Gase verändert. Betrachtet wird insbesondere eine gassensitive Schicht aus Wolframtrioxid und Titandioxid mit einem Wolframtrioxidanteil ≥ 50 Gew.-%. Weiterhin werden Herstellungsverfahren behandelt.

Um eine steigende Umweltbelastung durch die Verwendung fossiler Brennstoffe zu vermindern sucht man nach neuen Technologien um eine optimale Abgasreinigung zu erreichen. Eine Möglichkeit besteht in der Verwendung eines Entstickungskatalysators, der aus Titan/Wolfram-Mischoxiden aufgebaut ist und der künftig nicht nur in Verbrennungskraftwerken, sondern auch in Dieselmotoren von Fahrzeugen zum Einsatz kommen soll. Der Katalysator arbeitet mit Hilfe eines Reduktionsmittels, üblicher Weise Harnstoff, und ermöglicht eine Minderung des Stickoxidausstoßes um bis zu 98 %. Die Funktionsweise eines geregelten Dieselkatalysators wird im folgenden erklärt. Mit Hilfe eines Hydrolysekatalysators wird der im Fahrzeug mitgeführte Harnstoff über verschiedene Zwischenstufen zu Ammoniak zerlegt.

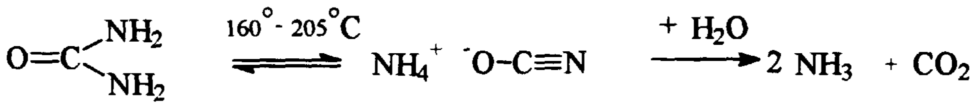


(1)

Harnstoff Ammoniumcyanat Ammoniak

Der so gewonnene Ammoniak wird dann mit den Stickoxiden im Entstickungskatalysator zu ungiftigem Stickstoff und Wasser umgesetzt.

$$4NH3 + 4NO + O_2 \rightarrow 4N_2 + 6H_2O \quad (2)$$

Um einen optimalen Wirkungsgrad dieses Verfahrens zu gewährleisten ist eine Sensorik erforderlich, die sowohl einen Stickoxid-Schlupf durch Unterdosierung wie auch einen Ammoniak-Schlupf durch Überdosierung des Reduktionsmittels detektieren kann. Die Detektion soll in Bezug auf $NO_x$ im Bereich um 100 ppm und bei Ammoniak um 50 ppm erfolgen.

In Otto-Motoren ist angedacht mit geeigneten Sensoren ebenfalls die Stickoxidemissionen hinter einem Dreiwege-Katalysator zu überwachen.

Im Stand der Technik wird die Harnstoffdosierung zur Reduktion der Stickoxide kennfeldgesteuert mit einer maximalen Konvertierungsrate von 70% betrieben. D.h. daß in der Motorsteuerung die Stickoxidkonzentrationen der verschiedenen Betriebspunkte als Kennfelddaten abgelegt sein müssen. Während der Fahrt wird dann so viel Harnstoff zudosiert, um rechnerisch 70 % der entstehenden Stickoxide zu konvertieren. Kennfeldgesteuert können keine höheren Konvertierungsraten erreicht werden, da es beispielsweise durch plötzliche Betriebspunktwechsel zu einem Ammoniakdurchbruch kommen könnte. Will man höhere Konvertierungsraten erhalten, muß die Stickoxidkonzentration vor und nach dem Katalysator beispielsweise im Konzentrationsbereich um 100 ppm mit Hilfe geeigneter Sensoren überwacht werden, so daß eine exakte Dosierung des benötigten Harnstoffes ermöglicht wird. Zusätzlich benötigt man nach dem Katalysator einen Sensor, der einen trotzdem auftretenden Ammoniakdurchbruch (MAK-Wert = 50 ppm) durch Überdosierung oder Katalysatordefekt detektieren kann.

Stickoxide können in gängigen Detektions- und Analyseverfahren mit Chemolumineszenz-Detektoren erkannt werden. Nachteil an diesen Verfahren ist die durch die benötigte Gasaufbereitung auftretende Zeitverzögerung, sowie der hohe Preis und die Größe des Meßgerätes, das eine Mitnahme in einem Kraftfahrzeug nicht ermöglicht. Der Einsatz von optischen Verfahren in Kraftfahrzeugabgasen ist aufgrund der hohen Verunreinigungen und des ungelösten Problems des "optischen Fensters" zur Zeit nicht vorstellbar.

Zur Detektion von Stickoxiden in Verbrennungsabgasen werden im wesentlichen zwei Sensorfunktionsprinzipien eingesetzt:

a) amperometrische und potentiometrische Sensoren auf der Basis von Sauerstoffionen leitendem $ZrO_2$. Diese werden mit unterschiedlich stark katalytisch aktiven Elektroden beschichtet. Die dadurch entstehende unterschiedliche Sauerstoffaktivität wird dann mittels des Sauerstoffionenleiters als Nernst-Spannung ausgegeben.

b) Leitfähigkeitssensoren auf der Basis von bei hohen Temperaturen halbleitenden Metalloxiden. Bei diesen wird die Leitfähigkeit eines halbleitenden Metalloxides gemessen, die sich aufgrund von Adsorptions- und Desorptionsvorgängen auf der Metalloxidoberfläche reversibel mit der Konzentration der zu detektierenden Stickoxide ändert.

In diesem Zusammenhang ist die deutsche Patentschrift 43 34 071 C1 zu nennen.

Weiterhin sind Sensoren bekannt, deren gassensitive Schichten beispielsweise aus Metalloxiden wie $MoO_3$ oder Molybdän-Wolframaten bestehen.

Bei der Suche nach optimalen Materialien für eine gassensitive Schicht eines Stickoxidgassensors wurde beispielsweise Wolframoxid ($WO_3$) oder Wolfram-Titan-Mischoxid untersucht. In diesem Zusammenhang sind die Literaturstellen [1] bis [6] zu nennen. Nur vereinzelt sind beim reinen Wolframoxid auch Untersuchungen zur Ammoniakdetektion bekannt [7]. Das größte Problem beim Wolframtrioxid ist die hohe Instabilität bei hohen Feuchtegehalten. Wolframtrioxid ($WO_3$) sublimiert ab ca. 800°C in Anwesenheit von Feuchte im Abgas [8,9] und die langsamen Ansprech- und Rückstellzeiten sind ebenfalls nachteilig. Einen kommerziellen Sensor auf der Basis von $WO_3$ -Dickschichten für niedrige Konzentrationen von $NO_2$ sind u.a. in den Literaturstellen [10] bis [12] zu finden. Derartige Sensoren dienen zur Erfassung von Dieselgeruch und Zigarettenrauch und können in Lüftungssteuerungen und Klimasystemen von Fahrzeugen und Gebäuden eingesetzt werden. Aufgrund seines Aufbaues in einem Plastikgehäuse und seiner Betriebstemperatur von maximal 400°C kann jedoch ein derartiger Sensor nicht ohne weiteres in den Abgastrakt eines Kraftfahrzeuges eingebracht werden, da dort Abgastemperturen von über 500°C möglich sind. Bezüglich eines eine gassensitive Schicht zu verwendenden Materials ist anzumerken, daß beispielsweise gesputtertes Wolframtrioxid-Titandioxid als Dünnschicht, sowie mechanisch gemischtes Wolframtrioxid-Titandioxid als Dickschicht und außerdem Kalzium oder Kupferwolframate durchwegs geringe Empfindlichkeiten sowohl auf Ammoniak als auch auf Stickoxide aufweisen. Dabei wurden bei den Dünnschichten Mischungsverhältnisse von 100:0, 90:10, 50:50, 10:90 und 0:100 untersucht. Auch das zur Herstellung eines Entstickungskatalysators verwendete Pulver, das zu 90% aus Titandioxid besteht und naßchemisch mit Wolframtrioxid (10%) präpariert wird, zeigt kaum Sensitivität auf Ammoniak. Die Sensitivität von Wolframtrioxid und Titandioxid auf Stickoxide bei tiefen Temperaturen wurde bereits erwähnt. Bei höheren Temperaturen läßt sich lediglich eine Ammoniaksensitivität feststellen. Die Untersuchung verschiedener Sensitivitäten muß die Stabilität und damit Einsatzfähigkeit einer gassensitiven Schicht berücksichtigen. Die mangelnde Langzeitstabilität der Sensorschichten wurde dadurch belegt, daß bei 200 Stunden Betrieb und einer Temperatur von 700°C eine 95%ige Degradation einer Wolframtrioxid-Dünnschicht zu verzeichnen ist.

Der Erfindung liegt die Aufgabe zugrunde, eine gassensitive Schicht und ein Herstellungsverfahren bereitzustellen, womit eine hohe Sensitivität auf NO, $NO_2$, $NH_3$ oder $CH_x$ einhergehend mit einer mechanischen und thermischen Beständigkeit gewährleistet ist.

Die Lösung dieser Aufgabe geschieht durch die in den Ansprüchen 1 bzw. 11 oder 12 enthaltene Kombination von Merkmalen.

Der Erfindung liegt die Erkenntnis zugrunde, daß durch eine Mischung aus Wolframtrioxid und Titandioxid mit einem überwiegenden Anteil von Wolframtrioxid bei gleichzeitiger Ausbildung des Wolframtrioxides als getragene Verbindung die notwendigen Anforderungen an eine Sensitivität und eine Stabilität in Bezug auf Phasenumwandlung oder Sublimation erzielt werden. Damit ist eine bei Wolframtrioxid bei der Verwendung im höheren Temperaturbereich ab beispielsweise 700°C vor allem in feuchter Gasatmosphäre auftretende Sublimation vermindert. Die Komponente Titandioxid verhindert die Sublimation und die Lebensdauer des Sensors wird verlängert. Durch den Einsatz einer getragenen Verbindung wird das Phänomen ausgenutzt, daß sich Oberflächenspezies, die sich nur an der Grenzschicht zum Träger ausbilden, vorteilhaft auf die Elektronenverteilung auswirken. Ihre Koordinationsgeometrie oder ihre Elektronenverteilung unterscheiden sich von den Strukturen im Volumen eines Kristalles, da sie sich an die Geometrie und an die elektronischen Zustände des Trägers anpassen. Bei der Verwendung von Wolframtrioxid als Sensormaterial für Ammoniak ergibt sich eine bessere Reduktionsfähigkeit und daher eine Erhöhung der Sensitivität, wenn Titandioxid als Trägermaterial verwendet wird. Untersuchungen haben gezeigt, daß ein Titanüberschuß in der gassensitiven Schicht langsamere Ansprechzeiten und geringere Sensitivitäten hervorruft. Aus diesem Grund werden für Sensoren mit schneller Ansprech- und Rückstellzeit und ausreichender Sensitivität gassensitive Schichten mit Wolframüberschuß vorgeschlagen.

Eine gassensitive Schicht gleicher chemischer Zusammensetzung für einen resistiven Gassensor kann auch Makromolekülen dargestellt werden, die ihre vorteilhafte Wirkung aufgrund erhöhter Oberfläche erzielen. Derartige Makromoleküle können beispielsweise in Sol-Gel-Verfahren dargesellt werden.

Im folgenden werden anhand von schematischen nicht einschränkenden Figuren Ausführungsbeispiele beschrieben.

Figur 1 zeigt für verschiedene Abgastemperaturen ein Sensorsignal für unterschiedliche Stickoxidmengen,
Figur 2 zeigt ein Sensorsignal für eine Ammoniakdetektion, aufgenommen im Zeitraum von einem halben Jahr,
Figur 3 zeigt einen Sensoraufbau mit einer entsprechenden Sensorschicht,
Figur 4 zeigt einen Sensoraufbau mit einer Schicht eines Oxidationskatalysators direkt auf der gassensitiven Schicht,
Figur 5 zeigt einen Sensoraufbau mit räumlich von der gassensitiven Schicht entkoppeltem Oxidationskatalysator

und

Figur 6 zeigt ein Regelungskonzept eines Entstickungssystemes.

Verfahren zur Herstellung einer gassensitiven Schicht nach einem der Ansprüche 1 bis 10 werden durch die Merkmalskombinationen von Anspruch 11 bzw. Anspruch 12 wiedergegeben.

Vorteilhafte Ausgestaltungen können den Unteransprüchen entnommen werden. Ein Verfahren zur Herstellung getragener Metalloxide besteht in der Imprägnierung des Trägers, in diesem Falle Titandioxid mit einer Lösung eines geeigneten Precursors (Vorstufe, Vorprodukt, Lösung) des entsprechenden Oxides (Wolframtrioxid $WO_3$). Im Falle des Wolframtrioxides kann man auf die Verbindung $A_2WO_4$ zurückgreifen, wobei A = H, Na, K, $NH_4$. Diese Verbindung löst sich in verdünntem Ammoniak oder Wasser. Bei der Präparation wird folgendermaßen vorgegangen: Der Ausgangsstoff $H_2WO_4$ mit einer Reinheit von mehr als 98% wird in Wasser suspendiert und unter Rühren mit so viel Ammoniak versetzt, daß sich der Feststoff gerade löst. Erwärmen beschleunigt den Lösungsvorgang. Anschließend wird festes $TiO_2$ (Modifikation Anatas mit einer Reinheit von > 99%) zugegeben. Die entstehende das feste $TiO_2$ enthaltende Suspension wird vorsichtig eingedampft. Die letzten Reste Feuchtigkeit werden in einem Trockenschrank bei etwa 120°C entfernt. Anschließend wird das Pulver kalziniert und ausführlich gemahlen, um Aglomerate zu zerstören und um eine homogene Ausgangssubstanz für eine Siebdruckpaste zu erhalten.

Eine andere Möglichkeit, ein gassensitives Material entsprechend der Erfindung zu erhalten, besteht in der Präparation nach dem Sol-Gel-Verfahren [14,15]. Dabei werden beide Oxide, Titandioxid und Wolframtrioxid, in einem Eintopfverfahren aus löslichen Vorstufen hergestellt. Als Vorstufen können beispielsweise verwendet werden $Ti(OC_3H_7)_4$ und $WCl_6$. Die Vorstufen werden in einem organischen Lösungsmittel gelöst und vorsichtig hydrolysiert. Die entstehende Molekühlstruktur stellt keinen Mischkristall dar und ist keine getragene Verbindung. Die beiden genannten Oxide fallen bei der Herstellung nebeneinander aus.

Zur Herstellung von Dickschichten wird das nach den obigen Verfahren entstandene Pulver mit einem organischen Binder angerührt, beispielsweise mit Terpinol, Ethylencellulose oder Dioctylphthalat. Nach dem Mischen wird die entstehende Paste in einem Siebdruckverfahren als dünne gassensitive Schicht dargestellt. Eine bevorzugte Schichtdicke beträgt beispielsweise 10 µm.

Der Figur 2 ist zu entnehmen, daß sich sowohl der Sensorgrundwiderstand als auch die Sensitivität eines Sensors nach der Erfindung nach einer Betriebsdauer von 4800 Stunden kaum verändern, was eine deutliche Verbesserung gegenüber den gesputterten Schichten bedeutet. Dies gilt sowohl bei der Zugabe von 75 als auch bei der Zugabe von 150 ppm $NH_3$. Die Sensortemperatur beträgt während der Messung 450°C, wobei sie bei der gesamten Betriebsdauer bei 550°C liegt.

Mit einem Sensor nach der Erfindung kann grundsätzlich Stickstoffmonoxid, Stickstoffdioxid, Ammoniak oder ein Kohlenwasserstoff detektiert werden. Um zu einem Gesamtgehalt von Stickoxiden zu kommen, wird dem Sensor ein in der Regel beheizter Katalysator vorgeschaltet. Dieser Katalysator ist zweckmäßigerweise ein Oxidationskatalysator. Seine Aufgabe besteht darin, durch bestimmte Gase verursachte Querempfindlichkeiten zu eliminieren. In diesem Sinne werden Kohlenwasserstoffe, Wasserstoff und Kohlenmonoxid zu Kohlendioxid und Wasser oxidiert. In Bezug auf die Stickoxide wird ein entsprechendes Verhältnis zwischen Stickstoffdioxid und Stickstoffmonoxid eingestellt, so daß die gesamte Stickoxidmenge detektierbar ist. Im Abgas einer Verbrennungskraftmaschine liegt ein ständig wechselndes NO/NO2-Gleichgewicht vor ($2NO+O_2 \Leftrightarrow 2NO_2$). Je nach Temperatur des Abgases, des Sensors bzw. des Katalysators und in Abhängigkeit von der Art des Katalysators, dem Sauerstoffpartialdruck usw. verschiebt sich das Gleichgewicht entweder auf die Seite des Stickstoffmonoxides oder des Stickstoffdioxides. Je nach Mengenverhältnis $NO/NO_2$ können dann entweder die reduzierenden (NO) oder die oxidierenden ($NO_2$) Eigenschaften des Meßgases vorherrschen und somit am Sensor eine Widerstandserniedrigung oder eine Widerstandserhöhung hervorrufen (siehe hierzu Figur 1). Bei einem ungünstigen $NO/NO_2$-Verhältnis können sich beide Signale kompensieren, so daß der Sensor keine Signaländerung zeigt.

Ein Oxidationskatalysator sollte unmittelbar vor der gassensitiven Schicht angebracht sein, so daß durch Aufoxidation entstandenes Stickstoffdioxid sich nicht wieder in Stickstoffmonoxid zurückverwandelt. Bei konstanter Arbeitstemperatur wandelt ein Oxidationskatalysator stets eine bestimmte Menge Stickstoffmonoxid in Stickstoffdioxid um. Ein auftretendes $NO_2$-Signal wird dann eine Aussage über den Gesamtstickoxidwert im Abgas machen und ermöglicht somit die genaue Steuerung der Ammoniakdosierung. Ein Oxidationskatalysator besteht meist aus einem rein keramischen oder einem metallischen Träger mit keramischer Beschichtung, versehen mit einer Edelmetallimprägnierung. Denkbar ist aber auch ein rein oxidischer Katalysator bestehend aus $TiO_2$ mit $V_2O_5$, mit CuO oder mit $MnO_2$. Der Oxidationskatalysator kann auf verschiedene Arten vor dem Sensor angebracht werden. Figur 3 zeigt zunächst einen Gassensor nach der Erfindung ohne einen Katalysator bzw. eine Katalysatorschicht. Figur 4 zeigt einen Stickoxidsensor mit einem direkt auf dem Sensor aufgebrachten Oxidationskatalysator. In den Figuren 3 und 4 ist die gassensitive Schicht mit 1 bezeichnet, die Struktur der elektrischen Heizung mit 2, die Meßelektrode mit 3, der Temperaturfühler mit 4 und der Oxidationskatalysator mit 5. Figur 5 zeigt eine weitere Darstellung eines Stickoxidsensors mit einem räumlich entkoppelten Oxidationskatalysator. In Figur 4 wird der Oxidationskatalysator als zusätzliche

Schicht direkt über der gassensitiven Schicht 1 aufgebracht. Vorteil dieses Aufbaues ist die einfache Technologie, beispielsweise Mehrfachsiebdruck, wobei der Oxidationskatalysator 5 durch eine geregelte Heizung des Sensors automatisch eine konstante Arbeitstemperatur erfahren kann. Der Oxidationskatalysator 5 erfüllt zusätzlich die Oxidation einer Schutzschicht. Die Variante nach Figur 4 zeigt einen von der gassensitiven Schicht 1 getrennten Oxidationskatalysator 5. Dieser ist als eine Schutzkappe ausgebildet. Der Vorteil dieses Aufbaus liegt darin, daß nicht auf mögliche Wechselwirkungen zwischen dem Oxidationskatalysator 5 und der gassensitiven Schicht zu achten ist. Der Aufbau wird damit unabhängig vom verwendeten gassensitiven Material. Ferner ist es nicht nötig, daß Sensor und Oxidationskatalysator 5 die gleiche optimale Arbeitstemperatur besitzen. Für die Regelung eines Entstickungskatalysators muß der Gehalt an hinter dem System entweichenden Stickoxiden bzw. bei einer Harnstoffüberdosierung der Ammoniak-Schlupf festgestellt werden. Mit einem Stickoxidsensor, der in Kombination mit einem Oxidationskatalysator arbeitet, kann der Gehalt an entweichendem Stickoxid, nicht aber ein eventueller Ammoniak-Schlupf festgestellt werden. Mit einer Regelstrategie entsprechend Figur 6, welche die Vorgeschichte und das bekannte Systemverhalten einbezieht, ist diese Aufgabe jedoch uneingeschränkt lösbar. Mit einer Regelstrategie entsprechend Figur 6 kann jedoch unter Einbeziehung der Vorgeschichte und des bekannten Systemverhaltens eine Regelung eines Entstickungssystems durchgeführt werden, bei dem Ammoniak in Abhängigkeit vom Gehalt des entweichenden Stickoxides vordosiert wird. Der am Stickoxidsensor meßbare Widerstand wird überwacht. Fällt dieser Widerstand, so wird weiterhin Ammoniak dosiert. Fällt der Widerstand nicht, so wird die Ammoniakdosierung gestoppt. Im Anschluß daran wird kontrolliert, ob der Sensorwiderstand steigt. Ist dies der Fall, so wird weiter Ammoniak zudosiert. Steigt der Sensorwiderstand nicht, so wird die Ammoniakdosierung weiterhin gestoppt.

Literatur:

[1] G. Sbervegliere, L. Depero, S. Gropelli, P. Nelli:
$WO_3$ sputtered thin films for Nox monotoring,
Sensors and Actuators B26-27 (1995) S. 89-92.
[2] Hong-Ming Lin; Chi-Ming Hsu, Huey-Yih Yang, Pee-Yew Lee, Chao-Cheng Yang:
Nanocrystalline $WO_3$-based $H_2S$ sensors,
Sensors and Actuators B22 (1994) S. 63-68.
[3] M. Akiyana, J. Tamaki, N. Miura, N. Yamazoe:
Tungsten oxide-based semiconductor sensor highly sensitive to NO and $NO_2$,
Chemistry Letters, S. 1611-1614, 1991.
[4] T. Inoue, K. Ohtsuka, Y. Yoshida, Y. Matsuura, Y. Kajiyama: Metall oxidse semiconductor $No_2$ sensor,
Sensors and Actuators B24-25 (1995) S. 388-391.
[5] C. Cantalini, H.T. Sun, M. Faccio, M. Pelino, S. Santucci, M. Passacantando:
NO2 sensitivity of $WO_3$ thin film obtained by high vacuum thermal evaporation,
Sensors and Actuators B31 (1996) S. 81-87.
[6] B. Lemire:
Gassensitive elektrische Eigenschaften von $WO_3$-Dünnschichten;
Dissertation TU München, 1994.
[7] T. Maekawa, J. Tamaki, N. Miura, N. Yamazoe:
Chemistry Letters, S. 639-642, 1992.
[8] GMELIN, Band W, S. 54.
[9] Zeitschrift für anorganische und allgemeine Chemie,
Bd. 316, Heft 3-4, S. 168, 1962.
[10] TGS 822 der Firma Figaro.
[11] Robert Bosch GmbH: Sensor zum Nachweis von Stichoxid,
Aktenzeichen PCT/DE94/01051, 14.09.1994
[12] Capteur Sensors & Analysers LTD: A gas sensor, Aktenzeichen PCT/GB92/00897 vom 18.05.1992
[13] DE 197 13 168 A1
[14] Ullman's Encyclopedia of Industrial Chemistry,
Vol. A14, S. 248-250, Sol-Gel-Verfahren zur Herstellung anorganischer Polymere,
Vol. A6, S. 73, Sol-Gel-Verfahren zur Herstellung von Beschichtungen.

**Patentansprüche**

1. Resistiver Gassensor bestehend aus einer gassensitiven Schicht, einer damit korrespondierenden Messelektrodenstruktur und einer Heizung, wobei die gassensitive Schicht aus einem Gemisch aus Wolframtrioxid ($WO_3$) und Titandioxid ($TiO_2$) besteht, in welchem der Anteil von Wolframtrioxid ($WO_3$) ≥ 50 Gew.-% ist und das Wolframtrioxid in getragener Form vorliegt, so daß Wolframtrioxid jeweils um einen Kern aus Titandioxid kristallisiert ist.

**2.** Resistiver Gassensor bestehend aus einer gassensitiven Schicht, einer damit korrespondierenden Messelektrodenstruktur und einer Heizung, wobei die gassensitive Schicht aus einem Gemisch aus Wolframtrioxid ($WO_3$) und Titandioxid ($TiO_2$) besteht, in welchem der Anteil von Wolframtrioxid ($WO_3$) ≥ 50 Gew.-% ist und das Gemisch durch in einem Sol-Gel-Verfahren hergestelle Makromoleküle mit erhöhter Oberfläche dargestellt ist.

**3.** Resistiver Gassensor nach einem der vorhergehenden Ansprüche wobei die gassensitive Schicht eine Stärke von 5 bis 50 μm aufweist.

**4.** Resistiver Gassensor nach einem der vorhergehenden Ansprüche, wobei dieser NO, $NO_2$, $NH_3$ oder $CH_x$ detektiert.

**5.** Resistiver Gassensor nach einem der vorhergehenden Ansprüche zur Detektion von $NO_2$, wobei dem Gassensor ein Oxidationskatalysator vorgeschaltet ist.

**6.** Resistiver Gassensor nach Anspruch 5, wobei der Oxidationskatalysator aus einem oxidischen Stoff wie $\gamma$-$Al_2O_3$, $SiO_2$ oder $TiO_2$ mit einer Edelmetallimprägnierung aus mindestens einem Edelmetall wie Pt, Rh, Pd oder Ir besteht oder als reiner oxidischer Katalysator aus $TiO_2$ mit $V_2O_5$, mit CuO oder mit $MnO_2$ dargestellt ist.

**7.** Resistiver Gassensor nach einem der Ansprüche 5 oder 6, wobei der Oxidationskatalysator als Deckschicht auf der gassensitiven Schicht dargestellt ist.

**8.** Resistiver Gassensor nach einem der Ansprüche 5 oder 6, wobei der Oxidationskatalysator als eine die gassensitive Schicht überdeckende und von dieser beabstandete Schicht dargestellt ist.

**9.** Resistiver Gassensor nach einem der vorhergehenden Ansprüche zur Regelung eines Entstickungssystemes.

**10.** Resistiver Gassensor nach Anspruch 9, wobei die Regelung die Dosierung von Ammoniak ($NH_3$) in Abhängigkeit vom Gehalt des entweichenden Stickoxides betrifft.

**11.** Verfahren zur Herstellung eines resistiven Gassensors nach einem der Ansprüche 1 oder 3-10, bestehend aus folgenden Schritten:

- ein fester Träger wird mit der Vorstufe von Wolframtrioxid, der Verbindung $A_2WO_4$, imprägniert, mit A=H, Na, K oder $NH_4$,
- die Vorstufe wird in Wasser suspendiert und unter Rühren mit Ammoniak (NH3) versetzt, so daß sich der Feststoff gerade löst,
- es wird festes Titandioxid (TiO2) zugegeben,
- die Suspension wird zur Herstellung eines Pulvers eingedampft und anschließend kalziniert.

**12.** Verfahren zur Herstellung einer gassensitiven Schicht für einen resistiven Gassensor nach einem der Ansprüche 2-10, wobei in einem Sol-Gel-Verfahren aus löslichen Vorstufen die Oxide Wolframtrioxid ($WO_3$) und Titandioxid ($TiO_2$) in einem Eintopfverfahren hergestellt werden, die Mischung in einem organischen Lösungsmittel gelöst ist und refluxiert, hydrolisiert und zur Herstellung eines Pulvers kalziniert wird.

**13.** Verfahren nach Anspruch 12, wobei als Vorstufen $Ti(OC_3H)_4$ und $WCl_6$ verwendet werden.

**14.** Verfahren nach einem der Ansprüche 12 oder 13, wobei zur Herstellung von Dickschichtsensoren das erhaltene Pulver mit einem organischen Binder angerührt und vermischt und die gassensitive Schicht mittels eines Siebdruckverfahrens dargestellt wird.

FIG 1

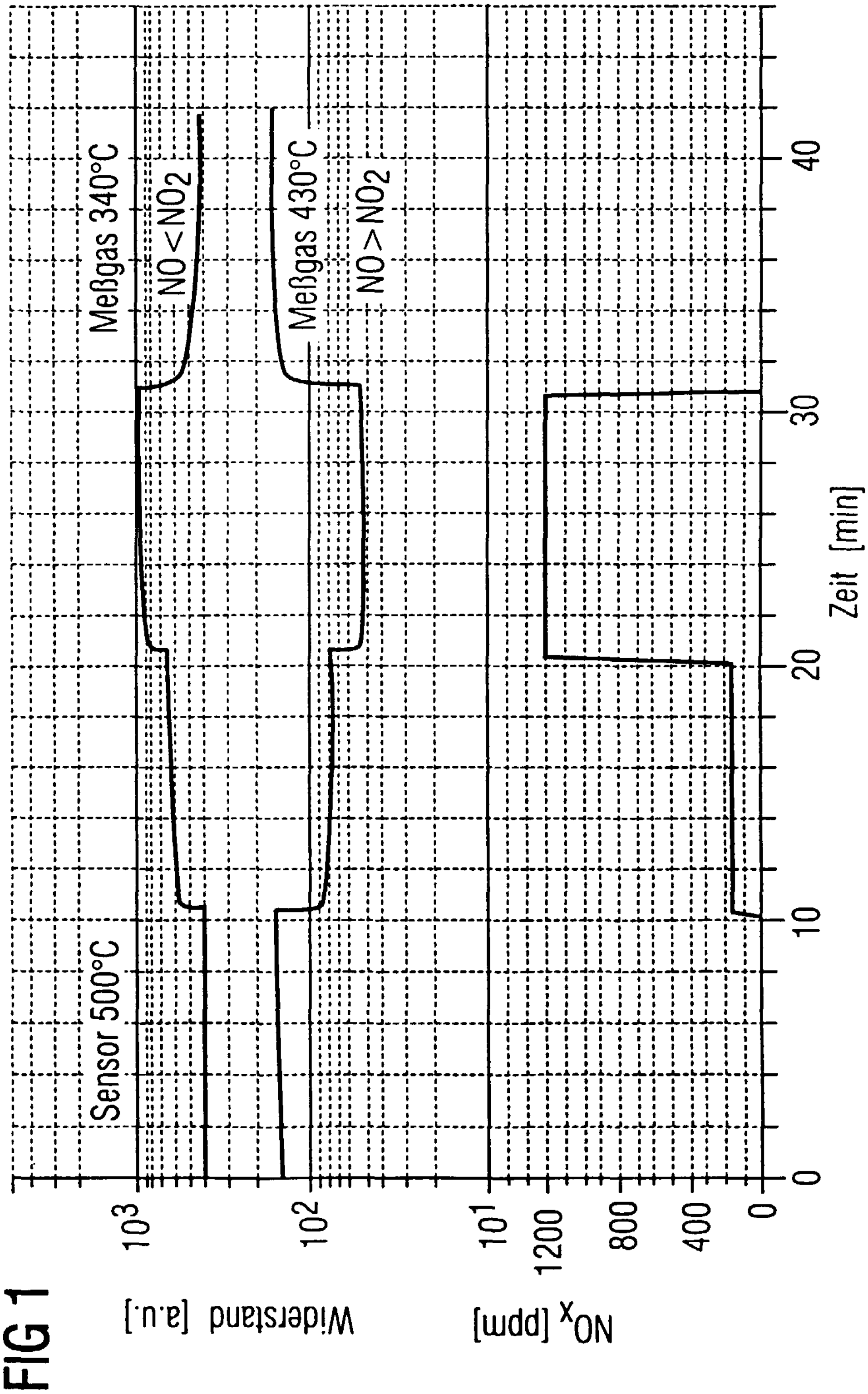
Widerstand [a.u.]
$NO_x$ [ppm]
$10^3$
$10^2$
$10^1$
1200
800
400
0
0
10
20
30
40
Zeit [min]
Sensor 500°C
Meßgas 340°C
$NO < NO_2$
Meßgas 430°C
$NO > NO_2$

FIG 2

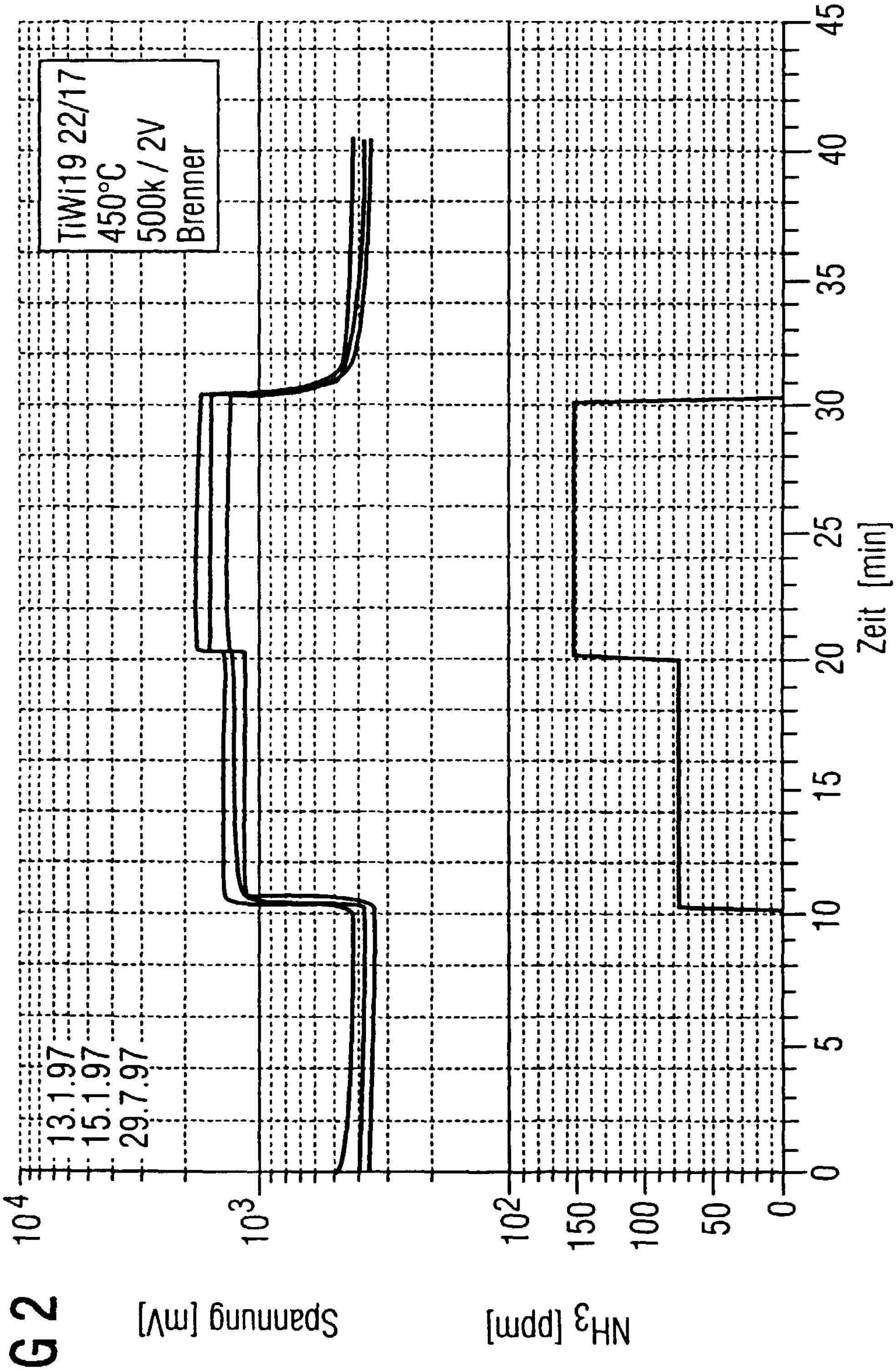
TiWi19 22/17
450°C
500k / 2V
Brenner
13.1.97
15.1.97
29.7.97
Spannung [mV]
$10^4$
$10^3$
$10^2$
NH$_3$ [ppm]
150
100
50
0
Zeit [min]
0
5
10
15
20
25
30
35
40
45

FIG 3

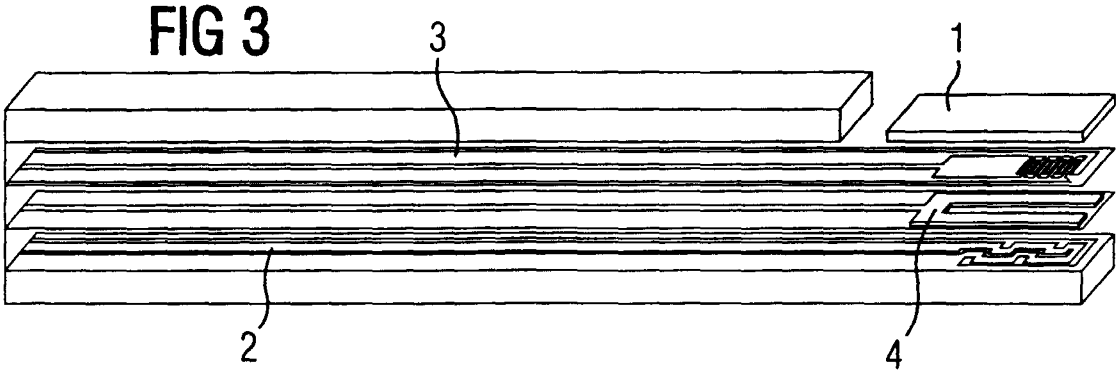


FIG 4

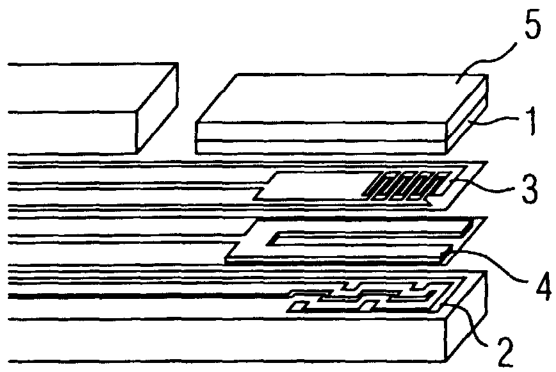


FIG 5

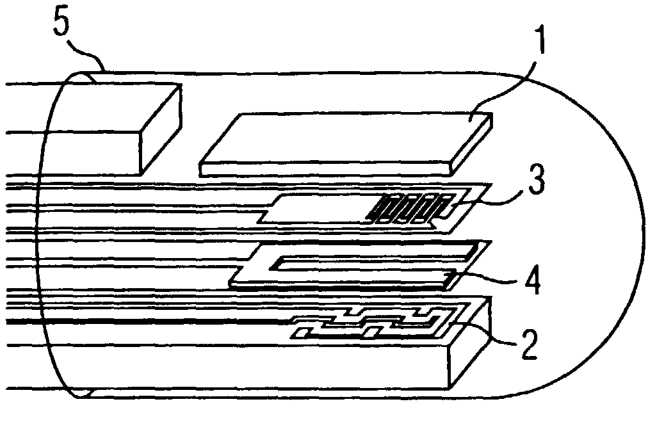

FIG 6

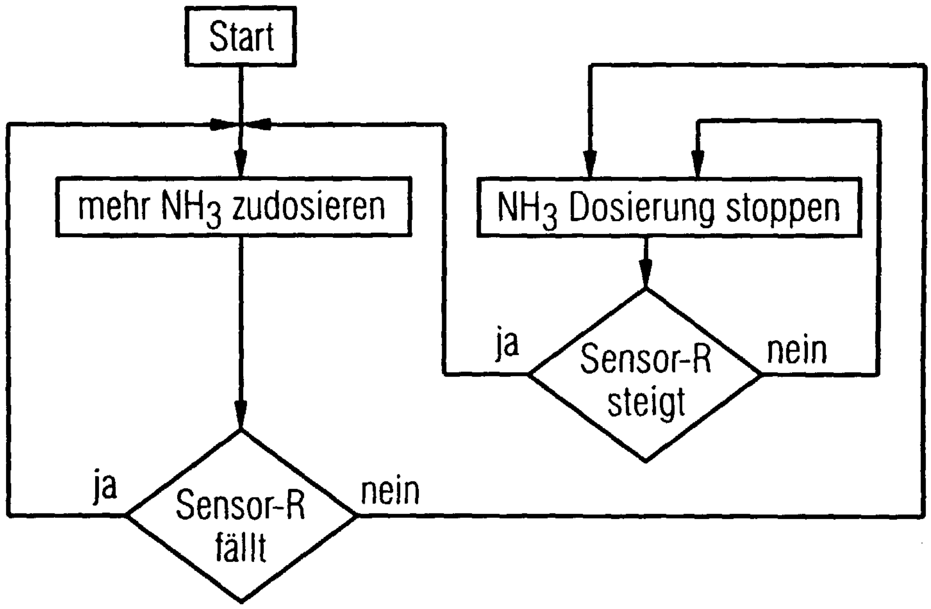
Start
mehr $NH_3$ zudosieren
$NH_3$ Dosierung stoppen
ja
Sensor-R steigt
nein
ja
Sensor-R fällt
nein